# EUROPÄISCHE PATENTSCHRIFT

(11) **EP 0 521 958 B1**
(45) Veröffentlichungstag und Bekanntmachung des Hinweises auf die Patenterteilung: **18.10.1995**
(21) Anmeldenummer: 91906588.8
(22) Anmeldetag: 15.03.1991
(51) Int. Cl.: C07C 57/03, C07C 51/353, C07C 69/587, C07C 67/347

(54) **VERFAHREN ZUR HERSTELLUNG VERZWEIGTER FETTSÄUREN UND DEREN ESTER**
PROCESS FOR PRODUCING BRANCHED FATTY ACIDS AND THEIR ESTERS
PROCEDE POUR LA FABRICATION D'ACIDES GRAS RAMIFIES ET DE LEURS ESTERS

(30) Priorität: 24.03.1990 DE 4009505
(43) Veröffentlichungstag der Anmeldung: 13.01.1993
(73) Patentinhaber: Henkel Kommanditgesellschaft auf Aktien, 40191 Düsseldorf (DE)
(72) Erfinder: NEUSS, Michael, Dr., D-5000 Köln 50 (DE); EIERDANZ, Horst, Dr., D-4010 Hilden (DE)
(86) Internationale Anmeldenummer: EP9100496
(87) Internationale Veröffentlichungsnummer: WO9114670

(56) Entgegenhaltungen:
- DE-A- 1 545 411
- DE-B- 1 140 187
- DE-C- 638 756
- Fette-Seifen-Anstrichmittel, Jahrgang 72, Nr. 8, 2970,M.J.A.M. den Otter : "The dimerization of oleic acid with a montnorillonite catalyst I: important process parameters; some main reactions", siehe das ganze Dokument in der Anmeldung erwähnt.

## Beschreibung

Gegenstand der Erfindung ist ein Verfahren zur Herstellung von verzweigten Fettsäuren und deren Estern durch Umsetzung von ungesättigten Fettsäuren und deren Ester mit aliphatischen, nicht-aktivierten Olefinen in Gegenwart von Schichtsilicaten und Aktivkohle.

Verzweigte Fettsäuren zeichnen sich gegenüber linearen Fettsäuren durch niedrigere Stockpunkte, geringere Flüchtigkeit, bessere Oxidationsstabilität, höheres Netzvermögen und leichtere Konfektionierbarkeit aus. Sie stellen damit wichtige Rohstoffe für die Herstellung von oberflächenaktiven Mitteln, beispielsweise Tensiden, Schmierstoffen, Walz- und Ziehölen, Textilhilfsmitteln und Kosmetika dar.

Eine Möglichkeit verzweigte Fettsäuren und deren Ester herzustellen, stellt die Guerbet-Reaktion [Soap,Cosm.Chem.Spec., 52, (1987)] dar, die über eine Alkoholdimerisierung beta-verzweigte Alkohole liefert, die ihrerseits durch Oxidation zu alpha-verzweigten Säuren umgesetzt werden können. Dieses Verfahren ist jedoch präparativ aufwendig.

Ein seit langem bekanntes Verfahren zur Herstellung verzweigter Fettsäuren besteht in der thermischen Behandlung von ungesättigten Fettsäuren, insbesondere Tallölfettsäure, mit Montmorillonit und Wasser oder homogenen Rutheniumkatalysatoren [Fette, Seifen, Anstrichm., **72,** 667 (1970), J.Am.Oil.Chem.Soc., **56,** 782 (1979), Rev.Fr.Corps.Gras **33,** 431 (1986)]. Das dabei anfallende Reaktionsgemisch enthält jedoch im wesentlichen hochmolekulare Dimer-, Trimer- und Tetramerfettsäuren, während der Anteil verzweigter Monomerfettsäuren, z. B. Isostearinsäure, vergleichsweise gering ist und nur mit hohen technischen Aufwand aus dem Gemisch abgetrennt werden kann. Für eine wirtschaftliche Herstellung von verzweigten Fettsäuren kommt die Fettsäuredimerisierung somit nur eingeschränkt in Betracht.

Aus der amerikanischen Patentschrift US 2,361,018 (1944) ist bekannt, daß sich trocknende Öle mit aktivierten Olefinen, z. B. Cyclopentadien, Dicyclopentadien oder Inden zu verzweigten Fettsäuren umsetzen lassen, die Anwendung in der Lackindustrie finden. Über die Diels-Alder- und En-Reaktionen von ungesättigten Fettsäuren und weiteren aktivierten Olefinen wie z. B. Acrolein, Acrylsäure, Crotonaldehyd, Crotonsäure, Maleinsäureanhydrid, Maleinsäure oder Methylvinylketon wird in Fette, Seifen, Anstrichm., **63**, 633 (1961) und Fat Sci.Techn., **90,** 1 (1988) berichtet. Die Umsetzung von Butadien mit ungesättigten Fettsäuren ist in J.Chem. Soc.Chem.Comm., **7,** 251 (1974) beschrieben. Gemäß der Lehre der japanischen Offenlegungsschrift JP 53/34708 kann Erucasäureester mit Ethylen in Gegenwart von Wolframhexachlorid und aluminiumorganischen Verbindungen umgesetzt werden. Alle genannten Verfahren lassen sich jedoch nur mit hohem technischen Aufwand durchführen.

Ein einfaches Verfahren zur Herstellung von verzweigten Fettsäuren und deren Estern ist schließlich aus Fette, Seifen, Anstrichm. **65,** 105 (1963) bekannt. Hiernach lassen sich ungesättigte Fettsäuren mit kurzkettigen C₂₋₄-Olefinen in einer Dien-Reaktion umsetzen.

Das Verfahren läuft jedoch nur bei Einsatz von Ethylen und Propylen mit zufriedenstellenden Ausbeuten ab. Mit zunehmender Kettenlänge sowohl der ungesättigten Fettsäuren, als auch des Olefins, werden so geringe Ausbeuten erhalten, daß das Verfahren für eine technische Verwendung ebenfalls nicht in Betracht kommt.

Der Erfindung lag somit die Aufgabe zugrunde, ein Verfahren zur Herstellung verzweigter Fettsäuren und deren Ester zu entwickeln, das frei von den geschilderten Nachteilen ist.

Gegenstand der Erfindung ist ein Verfahren zur Herstellung von verzweigten Fettsäuren und deren Ester durch Umsetzung von ungesättigten Fettsäuren oder deren Ester mit aliphatischen, nichtaktivierten Olefinen in Gegenwart von Schichtsilicaten und Aktivkohle.

Es wurde überraschenderweise gefunden, daß die Umsetzung von ungesättigten Fettsäuren mit aliphatischen, nicht-aktivierten Olefinen in kurzen Zeiten mit vergleichsweise hohen Ausbeuten erfolgt. Die Erfindung schließt ferner die Erkenntnis ein, daß die Umsetzung in Gegenwart von Schichtsilicaten und Aktivkohle zu deutlich geringeren Anteilen an oligomeren Nebenprodukten führt, als dies nach dem Stand der Technik zu erwarten gewesen wäre.

Unter ungesättigten Fettsäuren sind aliphatische, lineare Carbonsäuren zu verstehen, die 16 bis 24 Kohlenstoffatome und 1 bis 5 Doppelbindungen aufweisen. Typische Beispiele hierfür sind Palmitoleinsäure, Elaidinsäure, Petroselinsäure, Linolensäure, Erucasäure, Arachidonsäure oder Clupanodonsäure. Aus Gründen der leichten Zugänglichkeit ist der Einsatz von ungesättigten Fettsäuren mit 1, 2 oder 3 Doppelbindungen, insbesondere von Ölsäure, 9,12-Linolsäure und 9,11- Linolsäure ("Konjuensäure") bevorzugt.

Wie in der Fettchemie üblich, können die ungesättigten Fettsäuren auch als technische Gemische vorliegen, wie sie bei der Spaltung natürlicher Fette und Öle, wie z. B. Korianderöl, Sojaöl, Baumwollsaatöl, Erdnußöl, Leinöl, Fischöl oder Rindertalg anfallen. Anteile an gesättigten Fettsäuren können in diesen Gemischen ebenfalls enthalten sein, sofern ihr Anteil an der Fettsäurezusammensetzung nicht mehr als 50 Gew.-% ausmacht. Aus Gründen des hohen Gehaltes an Öl- und Linolsäure sind Fettsäuregemische auf Basis von Rüböl und Sonnenblumenöl bevorzugt.

Die ungesättigten Fettsäuren können auch in Form ihrer Alkyl- oder Glycerinmono-, -di- oder -triester vorliegen. Unter Alkylestern sind hierbei Ester der ungesättigten Fettsäuren mit aliphatischen Alkoholen mit 1 bis 22 Kohlenstoffatomen und 0, 1, 2 oder 3 Doppelbindungen zu verstehen. Typische Beispiele hierfür sind Ethyl-, n-Propyl, i-Propyl, n-Butyl-, Pentyl-, Hexyl-, Octyl-, 2-Ethylhexyl-, Decyl, Lauryl-, Myristyl-, Cetyl-, Stearyl-, Oleyl-, Elaidyl-, Petroselinyl-, Linolyl, Linolenyl-, Behenyl oder Erucylester. Vorzugsweise werden jedoch Methylester eingesetzt.

Unter aliphatischen, nicht-aktivierten Olefinen sind lineare, cyclische oder verzweigte Alkene mit alpha- oder innenständiger Doppelbindung zu verstehen, die 5 bis 20 Kohlenstoffatome beinhalten. Typische Beispiele hierfür sind Penten-1, Hexen-1, Cyclohexen, Hepten-1, Octen-1, Cycloocten, Nonen-1, Decen-1, Undecen-1, Dodecen-1, Cyclododecen, Tetradecen-1, Hexadecen-1, Octadecen-1, Eicosen-1 sowie deren Doppelbindungs- und Gerüstisomeren. Eine besonders hohe Reaktivität weisen lineare alpha-Olefine mit 6 bis 18, insbesondere 8 bis 10 Kohlenstoffatomen auf, deren Einsatz bevorzugt ist.

Unter Schichtsilicaten sind im folgenden Salze der Elemente der 1. bis 3. Hauptgruppe des Periodensystems zu verstehen, die ein Kristallgitter mit jeweils in einer Ebene verketteten SiO₄-Tetraedern ("Schichtgitter") aufweisen [Ullmanns Enzyclopaedie der technischen Chemie, 4. Aufl. Bd. 21, 365 (1984)]. Typische Beispiele hierfür sind Talk Mg₃[(OH)₂|Si₄O₁₀] und Kaolinit Al₄[(OH)₈|Si₄O₁₀]. Als besonders wirksamer Katalysator hat sich Montmorillonit Al₂[(OH)₂|Si₄O₁₀] erwiesen, dessen Einsatz bevorzugt ist.

Das molare Einsatzverhältnis von ungesättigter Fettsäure oder deren Ester zu Olefin beträgt 1 : 0,5 bis 1 : 5, vorzugsweise 1 : 1 bis 1 : 3,5. Eine optimale Reaktionsgeschwindigkeit und ein geringer Anteil an Nebenprodukten wird beoabchtet, wenn man molare Verhältnisse von 1 : 2 bis 1 : 2,75 wählt.

Die Umsetzung der ungesättigten Fettsäuren oder deren Ester mit den Olefinen wird in Gegenwart von 1 bis 30, vorzugsweise 3 bis 15 Gew.-% Schichtsilicat, bezogen auf die Ausgangsstoffe Fettsäure oder Fettsäureester plus Olefin , durchgeführt. Eine besondere Selektivität zur Bildung von monomeren verzweigten Fettsäuren wird bei Einsatz von 5 - 10 Gew.-% Schichtsilicat beobachtet.

Die Umsetzung der ungesättigten Fettsäuren oder deren Ester mit Olefinen wird in gleichzeitiger Gegenwart von 0.01 bis 3 Gew.-% Aktivkohle, bezogen auf die Ausgangsstoffe durchgeführt. Eine besondere Selektivität zur Bildung von monomeren verzweigten Fettsäuren wird bei Einsatz von 0.1 bis 1 Gew.-% Aktivkohle beobachtet.

Die Umsetzung der ungesättigten Fettsäuren oder deren Ester mit Olefinen in Gegenwart von Schichtsilicaten und Aktivkohle wird bei Temperaturen von 200 bis 280°C durchgeführt. Für eine optimale Reaktionsgeschwindigkeit ist es vorteilhaft, die Umsetzung bei 220 bis 260°C durchzuführen. Insbesondere bei Einsatz niedrigsiedender Olefine empfiehlt es sich, die Reaktion im Autoklaven unter autogenem Druck durchzuführen.

Die Umsetzung der ungesättigten Fettsäuren oder deren Ester mit Olefinen in Gegenwart von Schichtsilicaten und Aktivkohle wird über einen Zeitraum von 0,1 bis 5 h durchgeführt. Insbesondere bei Einsatz von Octen-1 und Decen-1 werden die verzweigten Fettsäuren und deren Ester schon nach 0,25 bis 1,5 h in hohen Ausbeuten erhalten.

Nach beendeter Umsetzung lassen sich die verzweigten Fettsäuren durch Destillation gewinnen.

Die verzweigten Fettsäuren eignen sich zur Herstellung von oberflächenaktiven Mitteln, so z. B. von Tensiden, Schmierstoffen, Walz- und Ziehölen, Textilhilfsmitteln und Kosmetika.

Die folgenden Beispiele sollen den Gegenstand der Erfindung näher erläutern.

### Beispiele

### 1. Ausgangsstoffe

A1. Technische Sonnenblumenfettsäure, Edenor^{(R)} SBO5, Fa.Henkel
   - Iodzahl: : 130
   - Mittleres Molgewicht: : 280
   - Zusammensetzung: : 6 Gew.-% Palmitinsäure 4 Gew.-% Stearinsäure 28 Gew.-% Ölsäure 62 Gew.-% Linolsäure
A2. Technische Ölsäure, Edebor^{(R)} TiO₅, Fa.Henkel
   - Iodzahl: : 95
   - Mittleres Molgewicht: : 280
   - Zusammensetzung: : 3 Gew.-% Myristinsäure 6 Gew.-% Palmitinsäure 5 Gew.-% Palmitoleinsäure 3 Gew.-% Stearinsäure 73 Gew.-% Ölsäure 11 Gew.-% Linolsäure
A3. Technisches Sonnenblumenöl
   - Iodzahl: : 131
   - Mittleres Molgewicht: : 886
   - Zusammensetzung der Fettsäurekomponente: : 6 Gew.-% Palmitinsäure 4 Gew.-% Stearinsäure 28 Gew.-% Ölsäure 62 Gew.-% Linolsäure
B1. Octen-1 (97 gew.-%ig), Fa. Janssen Chimica Mittleres Molgewicht : 112
B2. Decen-1 (96 gew.-%ig), Fa. Janssen Chimica Mittleres Molgewicht : 140
C. Montmorrillonit K 10, Fa.Südchemie
D. Aktivkohle, gepulvert, reinst, Fa.Merck

### Beispiel 1 bis 3:

**Allgemeine Vorschrift zur Umsetzung von ungesättigten Fettsäuren mit Olefinen.** In einem 1-l-Rührautoklaven wurden 1 Mol Fettsäure A1 bzw. A2 und 2 - 2,75 Mol Olefin B1 bzw. B2 vorgelegt und mit 5 - 10 Gew.-% Montmorillonit und 0.1 Gew.-% Aktivkohle, beides bezogen auf die Ausgangsstoffe, versetzt. Die Reaktionsmischung wurde auf T = 240 - 260°C aufgeheizt und t = 0.25 - 1,5 h gerührt. Nach Beendigung der Reaktion wurde auf 20°C abgekühlt und die Feststoffe über eine Nutsche abgetrennt.

Das Filtrat wurde anschließend im Hochvakuum bei 40 Pa und 20 bis 240°C destilliert. Bei einer Brüdentemperatur von 20 - 195°C destillierte zunächst ein Vorlauf über, der im wesentlichen überschüssiges Olefin und dessen Oligomerisierungs- und Isomerisierungsprodukte sowie geringe Anteile Fettsäure enthielt und verworfen wurde. Die verzweigten Fettsäuren fielen bei einer Brüdentemperatur von 195 - 240°C als hellgelbe Flüssigkeit an und wurden abgetrennt. Im Sumpf verblieben Anteile oligomerisierter Fettsäuren in Form einer dunkelbraunen, viskosen Flüssigkeit.

Die Reaktionsparameter und die nach Destillation erhaltenen Ausbeuten sind Tab.1 zu entnehmen.

### Beispiel 4:

**Umsetzung von Sonnenblumenöl mit Octen-1.** Beispiel 1 - 3 wurde unter Einsatz von 886 g (1 Mol) Sonnenblumenöl (A3), 924 g (8,25 Mol) Octen-1, 10 Gew.-% Montmorillonit und 0,1 Gew.-% Aktivkohle, beides bezogen auf die Ausgangsstoffe, wiederholt. Die Reaktion wurde bei T = 260°C über t = 1,5 h durchgeführt.

Die Destillation erfolgte nach Druckspaltung der Triglyceride analog zu den Beispiele 1 - 3.

**Tab.1**

| Reaktionsparameter und Ausbeuten | | | | | | | |
|---|---|---|---|---|---|---|---|
| Bsp. | Fettsäure (-ester) | Olefin | Olefin Mol | Mont. Gew.-% | T °C | t min | Ausb.: Mol.-% |
| 1.1 | A1 | B1 | 2,00 | 5,0 | 260 | 1,50 | 34,2 |
| 1.2 | A1 | B1 | 2,75 | 5,0 | 260 | 1,50 | 40,0 |
| 1.3 | A1 | B1 | 2,00 | 10,0 | 260 | 1,50 | 35,8 |
| 1.4 | A1 | B1 | 2,00 | 10,0 | 240 | 1,50 | 33,3 |
| 1.5 | A1 | B1 | 2,00 | 10,0 | 260 | 0,25 | 27,6 |
| 2.1 | A1 | B2 | 2,00 | 10,0 | 260 | 1,50 | 31,7 |
| 3.1 | A2 | B1 | 2,00 | 10,0 | 260 | 1,50 | 33,4 |
| 4.1 | A3 | B1 | 2,00 | 10,0 | 260 | 1,50 | 32,1 |
| Legende: Mont. = Montmorillonit Ausb. = Molare Ausbeute an verzweigten Fettsäuren bezogen auf die molare Einsatzmenge an ungesättigter Fettsäure bzw. deren Ester | | | | | | | |

## Patentansprüche

1. Verfahren zur Herstellung von verzweigten Fettsäuren und deren Estern, dadurch gekennzeichnet, daß man ungesättigte Fettsäuren oder deren Ester mit aliphatischen, nicht-aktivierten Olefinen in Gegenwart von Schichtsilicaten und Aktivkohle umsetzt.

2. Verfahren nach Anspruch 1, dadurch gekennzeichnet, daß ungesättigte Fettsäuren mit 16 bis 24 Kohlenstoffatomen und 1, 2 oder 3 Doppelbindungen eingesetzt werden.

3. Verfahren nach Anspruch 1, dadurch gekennzeichnet, daß Ester von ungesättigten Fettsäuren mit 16 bis 24 Kohlenstoffatomen und 1, 2 oder 3 Doppelbindungen mit aliphatischen Alkoholen mit 1 bis 22 Kohlenstoffatomen und 0, 1, 2 oder 3 Doppelbindungen oder Glycerin eingesetzt werden.

4. Verfahren nach mindestens einem der Ansprüche 1 bis 3, dadurch gekennzeichnet, daß Olefine mit 5 bis 20 Kohlenstoffatomen eingesetzt werden.

5. Verfahren nach mindestens einem der Ansprüche 1 bis 4, dadurch gekennzeichnet, daß als Schichtsilicat Montmorillonit eingesetzt wird.

6. Verfahren nach mindestens einem der Ansprüche 1 bis 5, dadurch gekennzeichnet, daß die ungesättigten Fettsäuren bzw. deren Ester und die Olefine im molaren Verhältnis von 1 : 0,5 bis 1 : 5 eingesetzt werden.

7. Verfahren nach mindestens einem der Ansprüche 1 bis 6, dadurch gekennzeichnet, daß die Umsetzung in Gegenwart von 1 bis 30 Gew.-% Schichtsilicat, bezogen auf die Ausgangsstoffe, durchgeführt wird.

8. Verfahren nach mindestens einem der Ansprüche 1 bis 7, dadurch gekennzeichnet, daß die Umsetzung in Gegenwart von 0,01 bis 3 Gew.-% Aktivkohle, bezogen auf die Ausgangsstoffe, durchgeführt wird.

9. Verfahren nach mindestens einem der Ansprüche 1 bis 8, dadurch gekennzeichnet, daß die Umsetzung bei Temperaturen von 200 bis 280°C durchführt wird.

10. Verfahren nach mindestens einem der Ansprüche 1 bis 9, dadurch gekennzeichnet, daß die Umsetzung über einen Zeitraum von 0,1 bis 5 h durchführt wird.

## Claims

1. A process for the production of branched fatty acids and esters thereof, characterized in that unsaturated fatty acids or esters thereof are reacted with aliphatic, non-activated olefins in the presence of layer silicates and active carbon.

2. A process as claimed in claim 1, characterized in that unsaturated fatty acids containing 16 to 24 carbon atoms and 1, 2 or 3 double bonds are used.

3. A process as claimed in claim 1, characterized in that esters of unsaturated fatty acids containing 16 to 24 carbon atoms and 1, 2 or 3 double bonds with aliphatic alcohols containing 1 to 22 carbon atoms and 0, 1, 2 or 3 double bonds or glycerol are used.

4. A process as claimed in at least one of claims 1 to 3, characterized in that olefins containing 5 to 20 carbon atoms are used.

5. A process as claimed in at least one of claims 1 to 4, characterized in that montmorillonite is used as the layer silicate.

6. A process as claimed in at least one of claims 1 to 5, characterized in that the unsaturated fatty acids or esters and the olefins are used in a molar ratio of 1:0.5 to 1:5.

7. A process as claimed in at least one of claims 1 to 6, characterized in that the reaction is carried out in the presence of 1 to 30% by weight layer silicate, based on the starting materials.

8. A process as claimed in at least one of claims 1 to 7, characterized in that the reaction is carried out in the presence of 0.01 to 3% by weight active carbon, based on the starting materials.

9. A process as claimed in at least one of claims 1 to 8, characterized in that the reaction is carried out at temperatures of 200 to 280°C.

10. A process as claimed in at least one of claims 1 to 9, characterized in that the reaction is carried out over a period of 0.1 to 5 h.

## Revendications

1. Procédé pour la fabrication d'acides gras ramifiés et de leurs esters, caractérisé en ce que l'on fait réagir des acides gras insaturés ou leurs esters avec des oléfines aliphatiques non activées, en présence de silicates stratifiés et de charbon actif.

2. Procédé selon la revendication 1, caractérisé en ce que l'on met en oeuvre des acides gras insaturés comportant 16 à 24 atomes de carbone et 1, 2 ou 3 doubles liaisons.

3. Procédé selon la revendication 1, caractérisé en ce que l'on met en oeuvre des esters d'acides gras insaturés comportant 16 à 24 atomes de carbone et 1, 2 ou 3 doubles liaisons avec des alcools aliphatiques comportant 1 à 22 atomes de carbone et 0, 1, 2 ou 3 doubles liaisons ou de la glycérine.

4. Procédé selon au moins une des revendications 1 à 3, caractérisé en ce que l'on met en oeuvre des oléfines comportant 5 à 20 atomes de carbone.

5. Procédé selon au moins une des revendications 1 à 4, caractérisé en ce que l'on met en oeuvre de la montmorillonite comme silicate stratifié.

6. Procédé selon au moins une des revendications 1 à 5, caractérisé en ce que l'on met en oeuvre les acides gras insaturés ou leurs esters et les oléfines dans un rapport molaire de 1:0,5 à 1:5.

7. Procédé selon au moins une des revendications 1 à 6, caractérisé en ce que la réaction est opérée en présence de 1 à 30 % en poids de silicate stratifié, par rapport aux matières de départ.

8. Procédé selon au moins une des revendications 1 à 7, caractérisé en ce que la réaction est opérée en présence de 0,01 à 3 % en poids de charbon actif, par rapport aux matières de départ.

9. Procédé selon au moins une des revendications 1 à 8, caractérisé en ce que la réaction est opérée à des températures de 200 à 280 °C.

10. Procédé selon au moins une des revendications 1 à 9, caractérisé en ce que la réaction est opérée sur une période de 0,1 à 5 heures.
